# EUROPÄISCHE PATENTSCHRIFT

(11) **EP 3 319 907 B1**
(45) Veröffentlichungstag und Bekanntmachung des Hinweises auf die Patenterteilung: **05.08.2020**
(21) Anmeldenummer: 16733479.6
(22) Anmeldetag: 28.06.2016
(51) Int. Cl.: C09C 1/30, A61K 8/02, A61K 8/06, A61K 8/25, A61K 8/26, A61K 8/893, A61K 8/894, A61Q 19/00, A61Q 19/10, C01B 33/18, C08K 3/36, C08K 9/06, C09D 7/62, C09D 17/00, C08K 3/22

(54) **SIO2 ENTHALTENDE DISPERSION MIT HOHER SALZSTABILITÄT**
SIO2 CONTAINING DISPERSION WITH HIGH SALT STABILITY
DISPERSION CONTENANT SIO2 À STABILITÉ AU SEL ÉLEVÉE

(30) Priorität: 10.07.2015 EP 15176284
(43) Veröffentlichungstag der Anmeldung: 16.05.2018
(73) Patentinhaber: Evonik Operations GmbH, 45128 Essen (DE)
(72) Erfinder: LORTZ, Wolfgang, 63607 Wächtersbach (DE); FISCHER, Ulrich, 63776 Mömbris (DE); PANZ, Christian, 50389 Wesseling-Berzdorf (DE); BERGMANN, Gabriele, 63538 Grosskrotzenburg (DE)
(74) Vertreter: Evonik Patent Association
(86) Internationale Anmeldenummer: PCT/EP2016/064994
(87) Internationale Veröffentlichungsnummer: WO 2017/009035

(56) Entgegenhaltungen:
- US-A1- 2004 037 964
- US-A1- 2005 133 766
- US-A1- 2006 093 541
- US-A1- 2010 092 765
- US-A1- 2013 071 649

## Beschreibung

Die Erfindung betrifft SiO₂ enthaltende Dispersionen mit hoher Salzstabilität.

Die Verbesserung der Stabilität von wässerigen Siliciumdioxid-Dispersionen ist Gegenstand der Forschung. Gewöhnlich wird versucht über eine geeignete Oberflächenmodifizierung der Siliciumdioxidpartikel die Dispersion vor Sedimentation und Reagglomeration zu schützen.

So wird beispielsweise in US2004241101 eine stabile pharmazeutische Dispersion offenbart, die mit Polyethylenglykolen oberflächenmodifizierte Siliciumdioxidpartikel enthält. Letztere können beispielsweise erhalten werden, indem man ein mit Ammoniak stabilisiertes kolloidales Siliciumdioxid mit einem polyethoxylierten Trialkoxysilan zur Reaktion bringt.

Die US2002172827 beschäftigt sich unter anderem mit der Herstellung redispergierbarer, nanoskaliger Siliciumdioxidpartikel. Dabei wird ein negativ geladenes Silicasol mit einem Aluminiumoxid beschichtet. Nachfolgend wird als oberflächennmodifizierendes Mittel Natriumdodecylbenzolsulfonat zugegeben.

In WO2004035474 wird ein Verfahren zur Herstellung einer stabilen, wässrigen Dispersion beansprucht, die durch Mischen von silanisierten, kolloidalen Siliciumdioxidpartikeln mit einem organischen Bindemittel erhalten wird. Als Silanisierungsmittel dient beispielsweise ein Glycidylepoxysilan. Das organische Bindemittel kann ein Polyethylenglykol sein.

In Part. Part. Syst. Charact. 2014, Vol. 31(1), pp 94-100 werden kolloidale Siliciumdioxidpartikel mit 2-[Methoxy(polyethylenoxy)propyl]trimethoxysilan oberflächenmodifiziert um die Salzstablität zu erhöhen.

In der WO03/106339 wird eine Fällungskieselsäure beschrieben, die eine BET-Oberfläche von 150 - 400 m²/g, eine CTAB-Oberfläche von 140 - 350 m²/g und einen Al₂O₃-Gehalt von 0,2 - 5 Gew.-% aufweist. Diese Kieselsäure kann mit einer Vielzahl von Silanen modifiziert werden und zu hydrophilen als auch zu hydrophoben Produkten führen. Auch das Verhältnis Silan zu Fällungskieselsäure kann über weite Grenzen variiert werden, nämlich 0.5 bis 50 Teile Silan, bezogen auf 100 Teile Fällungskieselsäure. Die Reaktion kann in der Dispersion der Fällungskieselsäure erfolgen, mit anschließender Trocknung und Temperung. Bedingungen hierzu werden nicht genannt, die Eigenschaften der Dispersion nicht weiter spezifiziert. WO03/106339 offenbart lediglich gefällte Mischoxidpartikel mit Partikelgrößen von über 1 µm.

In WO02/22745 wird ein Verfahren zum Grundieren von Stahl offenbart, bei dem ein wässriges, Aluminiumoxid-Siliciumdioxid-Sol mit 0,05-2,0 Gew.-% Aluminiumoxid eingesetzt wird. Das Aluminiumoxid-Siliciumdioxid-Sol kann ein Silan-Kupplungsmittel enthalten, das Alkoxysilan-Gruppen und einen organischen Rest mit einer funktionelle Gruppe, wie eine Amino, Epoxid oder Isocyanat enthält. In WO02/22745 werden lediglich kolloidale Mischoxidpartikel mit Partikelgrößen von 12-22 nm offenbart.

In WO2010/042672 wird eine Beschichtungszusammensetzung für thermoplastische und duroplastische Substrate offenbart, umfassend eine wässrige Dispersion mit einem pH-Wert von weniger als 7,5. Diese enthält oberflächenmodifizierte Siliciumdioxid- und ggf. Aluminiumoxid-Nanoteilchen, mit einem mittleren Teilchendurchmesser von 40 nm oder weniger, ein Alkoxysilan-Oligomer und ein Silan-Kupplungsmittel. Als Oberflächenmodifizierungsmittel kommen solche in Frage, die einen Rest aufweisen, der mit den Silanolgruppen auf der Siliciumdioxidoberfläche reagieren kann, sowie einen hydrophilen Rest, beispielsweise ein Säurerest, eine Ammoniumrest, einen Polyoxyethylenrest oder eine Hydroxylgruppe. WO2010/042672 offenbart keine Dispersionen enthaltend pyrogene Metalloxidpartikel.

US2010/0092765A1 offenbart wässrige Dispersionen von mit Silanen oberflächenmodifizierten Silicananopartikel, die Aluminiumoxid enthalten können, mit mittleren Partikeldurchmesser von 40 nm oder kleiner. Es werden keine pyrogenen Metalloxidpartikeln offenbart.

US2005/133766A1 offenbart wässrige Dispersionen enthaltend wenigstens zwei chemisch unterschiedliche Partikeltypen mit einer mittleren Partikelgröße von kleiner 100 µm, die stabil gegenüber der Sedimentation sind. Dispersion von US2005/133766A1 kann unter anderen pyrogenen und oberflächenmodifizierten Kieselsäuren enthalten, wobei der Kohlenstoffgehalt solcher Partikel nicht offenbart wird. Die Dispersionen aus US2005/133766A1 können ein mechanisches Gemisch von Silica und Aluminiumdioxidpartikeln darstellen, wie in Beispiel 1 gezeigt. Allerdings, offenbart US2005/133766A1 keine Mischoxidpartikel.

Es hat sich jedoch gezeigt, dass für etliche Anwendungen die bisher erreichte Salzstabilität nicht ausreichend ist. Aufgabe der vorliegenden Erfindung war es daher, eine Dispersion bereitzustellen, die eine verbesserte Salzstabilität aufweist. Aufgabe der Erfindung war ebenso ein Verfahren zur Herstellung dieser Dispersion bereitzustellen.

Gegenstand der Erfindung ist eine wässerige Dispersion enthaltend ein oberflächenmodifiziertes, pyrogenes hydrophiles Mischoxidpulver umfassend Silicium und Aluminium, wobei
a) die Oberfläche der Partikel Si- und Al-Atome und
b) eine Oberflächenmodifizierung ein Si-Atom aufweist, welches über ein C-Atom an einen Kohlenwasserstoffrest gebunden ist und
c) der Kohlenstoffgehalt des oberflächenmodifizierten Mischoxidpulvers 3 - 25 Gew.-% beträgt und
d) das oberflächenmodifizierte Mischoxidpulver einen mittleren Partikeldurchmesser d₅₀ in der wässerigen Dispersion von 40 - 200 nm hat.

Die wässerige Dispersion ist definiert als aus fester und flüssiger Phase bestehend. In der flüssigen Phase können Bestandteile der Luft gelöst vorliegen.

Es ist wesentlich für die Erfindung, dass die Oberfläche der Partikel Si- und Al-Atome aufweist. Dispersionen mit Partikeln, die vor der Oberflächenmodifizierung eine Aluminiumoxidhülle aufweisen, bei denen die Oberfläche also nur Al-Atome aufweist oder solche, die nur aus Aluminiumoxid bestehen, weisen geringere Stabilitäten als die erfindungsgemäße wässerigen Dispersion auf.

Unter Mischoxidpulver soll eine innige Mischung der Mischoxidkomponenten Aluminium und Silicium auf atomarer Ebene verstanden werden, bei der die Partikel auch Si-O-Al-Bindungen aufweisen können.

Unter oberflächenmodifiziert ist zu verstehen, dass die Kieselsäure an ihrer Oberfläche Gruppen trägt, die den Partikeln weitestgehend die hydrophilen Eigenschaften erhält, die die nicht modifizierte Kieselsäure aufweist. Hierdurch bleibt die wässerige Dispersion stabil. Stabil soll heißen, dass keine nennenswerte Reagglomeration und damit keine Sedimentation erfolgen. Hydrophobierte Partikel würden in einer wässerigen Lösung in kürzester Zeit reagglomerieren und separieren.

Für die wässerige Dispersion der vorliegenden Erfindung gilt, dass eine 0,5 gewichtsprozentige wässerige Dispersion in einer, Seewasser simulierenden, Referenzlösung wenigstens einen Monat bei einer Temperatur von 60°C stabil ist. Die Testung der Stabilität erfolgt in einer Referenzlösung, die erhalten wird, indem man bei 23°C zu einem Gemisch aus 28,500 g NaCl, 0,220 g NaHCO₃, 4,066 g Na₂SO₄, 1,625 g CaCl₂ x 2 H₂O, 3,162 g MgCl₂ x 6 H₂O, 0,024 g SrCl₂ x 6 H₂O und 0, 721 g KCl so viel vollentsalztes Wasser gibt, bis man 1000 ml Lösung enthält.

In einer besonderen Ausführungsform weist das Mischoxidpulver bei der das Si-Atom, welches über ein C-Atom an einen Kohlenwasserstoffrest gebunden ist, zusätzlich -Si-O-Al-Bindungen auf, wobei das Al-Atom Bestandteil der Partikeloberfläche ist.
Eine geeignete analytische Methode zur Detektion der Oberflächenelemente und deren Bindungen ist die röntgeninduzierte Photoelektronen-Spektroskopie (XPS). Durch schrittweises Absputtern kann ein Tiefenprofil erzeugt werden. Zusätzliche Information über die Zusammensetzung der Oberfläche kann durch energiedispersive Röntgenstrahlung (TEM-EDX) bestimmt werden. Die Zusammensetzung des Gesamtpartikels kann durch chemische oder physikalisch-chemische Methoden, z.B. Röntgenfluoreszenzanalyse, bestimmt werden.

Die Anteile an AI und Si können im oberflächenmodifizierten Mischoxidpulver über weite Grenzen variiert werden. Als geeignetes Maß hat sich das Zetapotential der Dispersion erwiesen. Dieses Zetapotential sollte negativ sein.

Ausgedrückt als Gewichtsverhältnis der Oxide, ist ein Gewichtsverhältnis Al₂O₃/SiO₂ < 1, d.h. Al₂O₃ ist mit weniger als 50 Gew.-% die Nebenkomponente. Es hat sich gezeigt, dass ein Mischoxidpulver mit weit geringeren Anteilen an Aluminiumoxid zu einer besseren Stabilität der wässerigen Dispersion führt als Mischoxidpulver mit hohen Anteilen an Aluminiumoxid. Unter geringen Anteilen ist zu verstehen, dass das Gewichtsverhältnis Al₂O₃/SiO₂ im oberflächenmodifizierten Mischoxidpulver 0,1:99,9 - 5:95, besonders bevorzugt 0,2:99,8 - 3:97 ist.

Dabei kann das Gewichtsverhältnis Al₂O₃/SiO₂ an der Oberfläche größer, kleiner oder gleich dem Gewichtsverhältnis im Gesamtpartikel sein. Bevorzugt ist ein Verhältnis (Al₂O₃/SiO₂)_{Oberfläche}/ (Al₂O₃/SiO₂)ₜₜₗ von 0,1 - 10. "ttl." entspricht dem Gewichtsverhältnis im Gesamtpartikel. Das Gewichtsverhältnis auf der Oberfläche kann zum Beispiel durch röntgeninduzierte Photoelektronen-Spektroskopie (XPS) bestimmt werden. Das Gewichtsverhältnis im Gesamtpartikel kann durch chemische oder physikalisch-chemische Methoden, z.B. Röntgenfluoreszenzanalyse, bestimmt werden.

In der erfindungsgemäßen Dispersion liegen solche Mischoxidpartikel vor, die aus pyrogenen Prozessen erhalten werden. Dabei werden Silicium- und Aluminiumverbindungen in einer Flamme, erzeugt durch die Reaktion von Wasserstoff und Sauerstoff, umgesetzt. Die so erhaltenen Pulver werden als "pyrogen" oder "fumed" bezeichnet. Bei der Reaktion werden zunächst hochdisperse Primärpartikel gebildet, die im weiteren Reaktionsverlauf zu Aggregaten zusammenwachsen. Die Aggregatdimensionen dieser Pulver sind in der Regel im Bereich 0,2 - 1 µm.
Durch geeignete Vermahlung können diese in den für die vorliegende Erfindung vorteilhaften nm-Bereich überführt werden und nachfolgend mit einem Oberflächenmodifizierungsmittel behandelt werden.

Es hat sich gezeigt, dass die besten Ergebnisse bezüglich der Salz- und Temperaturstabilität der wässerigen Dispersion mit einem oberflächenmodifizierten Mischoxidpulver erhalten werden, welches in der Dispersion, einen mittleren Partikeldurchmesser d₅₀ von 40 - 200 nm aufweist. Der mittlere Partikeldurchmesser kann mit den üblichen dem Fachmann bekannten Methoden der Lichtstreuung zur Bestimmung von Teilchengrößenverteilungen in Dispersionen bestimmt werden.

Das oberflächenmodifizierte Mischoxidpulver kann in Form isolierter Einzelpartikel und/oder in Form von aggregierten Partikeln vorliegen. Im Falle von aggregierten Partikeln beschreibt der mittlere Partikeldurchmesser die Dimension des Aggregates.

Das in der erfindungsgemäßen wässerigen Dispersion vorliegende oberflächenmodifizierte Mischoxid ist unter anderem dadurch gekennzeichnet, dass die Oberflächenmodifizierung einen Kohlenwasserstoffrest umfasst, der über ein C-Atom an ein Si-Atom gebunden ist. Der Kohlenwasserstoffrest ist so zu wählen, dass das oberflächenmodifizierte Mischoxid in der wässerigen Dispersion hydrophile Eigenschaften aufweist. Dies ist beispielsweise von der Anzahl der Kohlenstoffatome des Kohlenwasserstoffrestes und dem Vorhandensein von funktionellen, hydrophilen Gruppen, wie Hydroxy-, Ether-, Amin- oder Carboxylgruppen abhängig. Bevorzugt ist der Kohlenwasserstoffrest durch ein oder mehrere Heteroatome unterbrochen. Besonders bevorzugt ist das Heteroatom O oder N.

Bevorzugt wird eine Oberflächenmodifizierung aus der Gruppe bestehend aus Si-(CH₂)ₙ-Yₘ-R ausgewählt, wobei Si das Si-Atom ist, welches über ein C-Atom an einen Kohlenwasserstoffrest gebunden ist, und
n = 1, 2, 3 und m= 0, 1;
für den Fall, dass m = 1 ist:
R = -H, -CH₃,-C₂H₅, -OH, -OCH₃, -OC₂H₅, -C(=O)OCH₃, -C(=O)OC₂H₅, -O-C(=O)CH₃,-O-C(=O)CH=CH₂, -O-C(=O)CH=CH(CH₃), -C(=O)CH₃, -C(=O)H, NH₂; 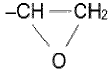 oder ein Gemisch der vorgenannten Reste R,
   und für den Fall, dass m=0 ist, R den vorgenannten Resten entspricht, jedoch ohne -H, -CH₃,-C₂H₅.
Y = -(OCR¹R²-CR³R⁴)ₒ-, o = 1 - 30, R¹, R², R³, R⁴ = unabhängig voneinander H oder CH₃, besonders bevorzugt o = 5 - 15 und R¹, R², R³, R⁴ = H;
   -(OCR¹R²-CR³R⁴-CR⁵R⁶)ₚ- , p = 1 - 30, R¹, R²,R³, R⁴,R⁵, R⁶= unabhängig voneinander H oder CH₃, -NHCH₂CH₂O-, -NH(CH₂)₂NH(CH₂)₂-, -NH(CH₂)₂NH(CH₂)₂- oder ein Gemisch der vorgenannten Reste Y.

Es ist ebenso denkbar, dass Y verzweigte Polyethylenglykole umfasst. Hierbei stellt R und wenigstens einer der Reste R¹-R⁶ eine -(OCH₂-CH₂)ᵣ-Gruppierung dar, mit r = 5 - 15.

In der erfindungsgemäßen wässerigen Dispersion beträgt der Anteil an Wasser bevorzugt 50 - 90 Gew.-% und an oberflächenmodifiziertem Mischoxidpulver bevorzugt 10 - 50 Gew.-%. Je nach der geplanten weiteren Verwendung kann der Anteil an oberflächenmodifizierten Mischoxidpulver weiter reduziert werden.

Es wurde gefunden, dass die flüssige Phase neben Wasser auch noch geringe Anteile von Alkohol, wie Methanol, Ethanol, Propanol oder Butanol enthalten kann. Der Anteil an Alkohol beträgt in der Regel weniger als ein 10 Gew.-%, bevorzugt 3 - 7 Gew.-%, jeweils bezogen auf die Dispersion.

Der pH-Wert der flüssigen Phase der wässerigen Dispersion beträgt bevorzugt 8 -12, besonders bevorzugt 9 - 11.

Die erfindungsgemäße wässerige Dispersion kann geringe Mengen, weniger als 100 ppm, an üblichen Dispergiermitteln enthalten. Die Anwesenheit von Dispergiermitteln ist im Rahmen der vorliegenden Erfindung jedoch nicht gewünscht. Der stabilisierende Effekt der erfindungsgemäßen wässerigen Dispersion liegt allein in dem oberflächenmodifizierten Silicium-Aluminium-Mischoxidpulver begründet.

Die Dispersion kann durch ein Verfahren hergestellt werden, bei dem man
ein Silicium und Aluminium umfassendes Mischoxidpulver, welches an der Oberfläche der Partikel jeweils Hydroxylgruppen tragende Si-Atome und Al-Atome aufweist in einem wässerigen Lösungsmittel dispergiert und nachfolgend
ein Mittel zur Oberflächenmodifizierung hinzufügt, bei dem ein Si-Atom über ein C-Atom an einen Kohlenwasserstoffrest und das Si-Atom weiterhin an ein oder mehrere Hydroxygruppen, Alkoxygruppen, Halogenidgruppen oder Mischungen daraus gebunden ist, und
das Gemisch zur Reaktion bringt und gegebenenfalls das Hydrolyseprodukt abtrennt und gegebenenfalls den pH-Wert einstellt,
wobei das Mittel zur Oberflächenmodifizierung in einer Menge hinzugefügt wird, um unter Berücksichtigung der bei der Hydrolyse abgespaltenen Hydroxygruppen, Alkoxygruppen oder Halogenidgruppen, einen Kohlenstoffgehalt von 3 - 25 Gew.-% zu erhalten.
Beim Einsatz von Verbindungen mit Alkoxygruppen ist ein Alkohol, beispielsweise Methanol oder Ethanol, das Hydrolyseprodukt.

Zur Dispergierung stehen dem Fachmann etliche Methoden zur Verfügung. Zur Herstellung feinteiliger Dispersionen stehen beispielsweise Vorrichtungen wie Ultraschallsonden, Kugelmühlen, Rührwerkskugelmühlen, Rotor-/Statormaschinen, Planetenkneter/-mixer oder Hochenergiemühlen oder Kombinationen zur Verfügung. So kann beispielsweise mittels eines Rotor-/Statorsystems eine Vordispersion hergestellt werden, die in einem nachfolgenden Schritt mittels einer Hochenergiemühle weiter vermahlen wird. Durch diese Kombination können beispielsweise extra feine Dispersionen mit einem Partikeldurchmesser von 200 nm oder weniger hergestellt werden. Bei einer Hochenergiemühle wird eine unter einem hohen Druck stehende Vordispersion in zwei oder mehrere Ströme geteilt, die dann über eine Düse entspannt werden und exakt aufeinandertreffen.

Es hat sich als vorteilhaft erwiesen, bereits von einer wässerigen Dispersion eines Silicium und Aluminium enthaltenden Mischoxidpulvers auszugehen.

In der Regel wird das Gemisch zur Reaktion gebracht, indem man einen pH-Wert von 11 oder mehr einstellt, das Gemisch bei einer Temperatur von 50 - 95°C über einen Zeittraum von 1 - 30 Minuten thermisch behandelt und nachfolgend gegebenenfalls einen pH-Wert von 8 - 10 einstellt.

Bei dem eingesetzten Silicium-Aluminium-Mischoxidpulver handelt es sich um eines, bei dem an der Oberfläche der Partikel jeweils Hydroxylgruppen tragende Si-Atome und Al-Atome vorliegen.

Es wird ein pyrogenes Silicium-Aluminium-Mischoxidpulver eingesetzt. Kommerziell erhältliche pyrogene Silicium-Aluminium-Mischoxidpulver sind AEROSIL® MOX 80 mit einer BET-Oberfläche von 60 - 100 m²/g und einem Aluminiumoxidanteil von 0,3 - 1,3 Gew.-% und AEROSIL® MOX 170 mit einer BET-Oberfläche von 140 - 200 m²/g und einem Aluminiumoxidanteil von 0,3 - 1,3 Gew.-%, beide Evonik Industries. Beide pyrogene Silicium-Aluminium-Mischoxidpulver können bevorzugt eingesetzt werden. Besonders bevorzugt ist AEROSIL® MOX 170.

Weiterhin kann das in EP-A-995718 offenbarte pyrogene Silicium-Aluminium-Mischoxidpulver eingesetzt werden. Es wird erhalten, indem man einen dampfförmigen Siliciumdioxid-Precursor und eine Aluminiumchloridlösung in einer Flamme zur Reaktion bringt. Durch die feine Verteilung von Aluminiumchlorid im Aerosol und während der Genese des Oxids in der Gasphase, erfolgt ein weitgehend homogener Einbau des Aluminiums.

Ebenso kann das in EP-A-2500090 offenbarte pyrogene Silicium-Aluminium-Mischoxidpulver, bei dem das Gewichtsverhältnis von (Al₂O₃/SiO₂)ₜₜₗ im Gesamtpartikel 0,002 bis 0,05, und das Gewichtsverhältnis (Al₂O₃/SiO₂)_{Oberfläche} der Partikel in einer oberflächennahen Schicht kleiner als im Gesamtpartikel ist, eingesetzt werden. Die Aluminiumoxidkonzentration an der Oberfläche ist also weiter reduziert.

Für das Verfahren wird das Mittel zur Oberflächenmodifizierung bevorzugt aus der Gruppe bestehend aus X₄₋ₐ[Si-(CH₂)ₙ-Yₘ-R]ₐ ausgewählt, mit
a = 1,2,3; bevorzugt a = 1; n = 1, 2, 3; m = 0, 1,
X = H, OH, OCH₃, OC₂H₅, OCH₂CH₂H₃, OCH(CH₃)₂; Cl,
für den Fall, dass m = 1 ist:
R = -H, -CH₃,-C₂H₅, -OH, -OCH₃, -OC₂H₅, -C(=O)OCH₃, -C(=O)OC₂H₅, -O-C(=O)CH₃, -O-C(=O)CH=CH₂, -O-C(=O)CH=CH(CH₃), -C(=O)CH₃, -C(=O)H, NH₂; 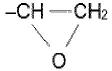 oder ein Gemisch der vorgenannten Reste R,
   und für den Fall, dass m=0 ist, R den vorgenannten Resten entspricht, jedoch ohne -H, -CH₃,-C₂H₅.
Y = -(OCR¹R²-CR³R⁴)ₒ-, o = 1 - 30, R¹, R²,R³, R⁴ = unabhängig voneinander H oder CH₃, besonders bevorzugt o = 5 - 15 und R¹, R²,R³, R⁴ = H;
   -(OCR¹R²-CR³R⁴-CR⁵R⁶)ₚ- , p = 1 - 30, R¹, R²,R³, R⁴,R⁵, R⁶= unabhängig voneinander H oder CH3, -NHCH₂CH₂O-, -NH(CH₂)₂NH(CH₂)₂-, -NH(CH₂)₂NH(CH₂)₂-, oder ein Gemisch der vorgenannten Reste Y ist.

Es ist ebenso denkbar, dass Y verzweigte Polyethylenglykole umfasst. Hierbei stellt R und wenigstens einer der Reste R¹-R⁶ eine -(OCH₂-CH₂)ᵣ-Gruppierung dar, mit r = 5 - 15.

Besonders bevorzugt kann das Mittel zur Oberflächenmodifizierung aus der Gruppe bestehend aus (CH₃O)₃Si(CH₂)₃-OCH₃, (CH₃O)₃Si(CH₂)₃-(OCH₂CH₂)₃-OCH₃, (CH₃O)₃Si(CH₂)₃-(OCH₂CH₂)₆₋₉-OCH₃, (CH₃O)₃Si(CH₂)₃-(OCH₂CH₂)₉₋₁₂-OCH₃, (CH₃O)₃Si(CH₂)₃-(OCH₂CH₂)₂₁₋₂₄-OCH₃ und (CH₃CH₂ O)₃Si(CH₂)₃-(OCH₂CH₂)₈₋₁₂OH ausgewählt sein.

Weiterhin kann das Mittel zur Oberflächenmodifizierung aus der Gruppe bestehend aus (RO)₃Si-(CH₂)₃-NH₂, (RO)₃Si-(CH₂)₃-CH-CH₂-NH₂, (RO)₃Si-(CH₂)₃-NH-(CH₂)₂-NH₂, (RO)₃Si-(CH₂)₃-NH-(CH₂)₂NH(CH₂)-NH₂, (RO)₃Si-(CH₂)₃-N-[(CH₂)₂NH(CH₂)-NH₂]₂, R= CH₃, C₂H₅, ausgewählt sein.

Weiterhin sind zur Oberflächenmodifizierung wässerige Zusammensetzungen geeignet, die Organopolysiloxane mit Glycidetheralkylresten, Acryloxyalkylresten und/oder Methacryloxyalkylresten enthalten. Weiterhin kann das Organopolysiloxan als weitere funktionelle Gruppen Aminoalkyl-Reste oder Alkyl-Reste oder Aminoalkyl- und Alkyl-Rest enthalten. Vorzugsweise trägt dabei jedes Silicium im Organopolysiloxan eine funktionelle Gruppe. Die organopolysiloxanhaltige Zusammensetzungen können erhalten werden durch Mischen wasserlöslicher Organosilane der Formel I

H₂N(CH₂)_{f}(NH)₉(CH₂)ᵢ-Si(CH₃)ₕ(OR)₃₋ₕ (I),

wobei 0 ≤ f ≤ 6, g = 0 falls f = 0, g =1 falls f >1, 0 ≤ i ≤ 6, 0 ≤ h ≤ 1 und R eine Methyl-, Ethyl-, Propyl- oder Isopropyl-Gruppe sind, bevorzugt Aminopropyltriethoxysilan,
mit
wasserlöslichen, jedoch in wässerigem Medium nicht stabilen Organosilanen der Formel II

X-CH₂O(CH₂)₃-Si(CH₃)ₕ(OR)₃₋ₕ (II),

wobei 0 ≤ h ≤1 ist und R einen Methyl-, Ethyl-, Propyl- oder Isopropyl-Rest darstellt, bevorzugt Glycidyloxypropyltrimethoxysilan und 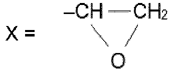 und/oder
Organosilanen der Formel III

H₂C=CR'-COO(CH₂)₃-Si(CH₃)ₕ(OR)₃₋ₕ (III),

wobei 0 ≤ h ≤ 1 ist, R einen Methyl-, Ethyl-, Propyl- oder Isopropyl-Rest und R' einen Methyl- oder Wasserstoff-Rest darstellen, bevorzugt Methacryloxypropyltrimethoxysilan,
und nicht wasserlöslichen Organosilanen der Formel IV

R"- Si(CH₃)ₕ(OR)₃₋ₕ (IV),

wobei 0 ≤ h ≤ 1 ist, R einen Methyl-, Ethyl-, Propyl- oder Isopropyl-Rest und R" einen linearen, verzweigten oder cyclischen Kohlenwasserstoff-Rest mit 1 bis 8 C-Atomen darstellen, bevorzugt Propyltrimethoxysilan, in dem molaren Verhältnis M = a/(b+c+d), wobei a die Summe der Molzahlen der Organosilane gemäß Formel I, b die Summe der Molzahlen der Organosilane gemäß Formel II sowie c die Summe der Molzahlen der Organosilane gemäß Formel III und d die Summe der Molzahlen der Organosilane gemäß Formel IV sind, mit 0 ≤ M ≤ 3 und mindestens b > 0 oder c > 0.

Das Gemisch wird mit einem Wasser/Säure-Gemisch versetzt, der pH-Wert der Reaktionsmischung auf einen Wert zwischen 1 und 8 eingestellt und der Alkohol entfernt.

Die organopolysiloxanhaltigen Zusammensetzungen können idealisiert dargestellt werden gemäß der Formel

HO[Si(A*)(OH)_{z}(CH₃)_{1-z}O]ₐ[Si(B*)(OH)_{y}(CH₃)_{1-y}O]_{b}[Si(C*)(OH)_{w}(CH₃)_{1-w}O]_{c}[Si(D*)(OH)ᵥ(CH₃)₁₋ᵥO]_{d}H(HX)ₑ (V)

wobei A* einen Aminoalkyl-Rest abgeleitet von der Formel I,
B* einen Glycidetheralkyl-Rest abgeleitet von der Formel II,
C* einen Acryloxyalkyl- oder Methacryloxyalkyl-Rest abgeleitet von der Formel III und
D* einen Alkyl-Rest gemäß der allgemeinen Formel IV bedeuten,
HX eine Säure darstellt, wobei X ein anorganischer oder organischer Säure-Rest ist,
v gleich 0 oder 1 und w gleich 0 oder 1 und y gleich 0 oder 1 und z gleich 0 oder 1 und a+b+c+d ≥ 4 und a ≤ e ≤ 2 a sind, mit 0 ≤ a/(b+c+d) ≤ 3, genügen.

Die organopolysiloxanhaltigen Zusammensetzungen weisen bevorzugt einen pH-Wert von 1 - 8, besonders bevorzugt von 3 - 6 auf.

Aus der erfindungsgemäßen wässerigen Dispersion kann durch Abtrennen der flüssigen Phase, beispielsweise durch Sprühtrocknung, ein leicht redispergierbares, oberflächenmodifiziertes Pulver erhalten werden. Dieses Pulver kann durch geringen Energieeintrag, beispielsweise durch Rühren, in eine wässerige Phase eingearbeitet werden, ohne dass es zu einer bemerkenswerten Aggregation der Partikel kommt. Der mittlere Partikeldurchmesser d₅₀ in dieser wässerigen Dispersion kann 40 - 200 nm betragen.

Die erfindungsgemäße wässerige Dispersion kann als Bestandteil von pharmazeutischen Zubereitungen, kosmetischen Zubereitungen, wasserbasierender Farbe und Lacke, von Reinigungsmitteln, von Geschirrspülmitteln und von Streichfarben in der Papierindustrie verwendet werden.

### Beispiele

### Salzstabilität bei erhöhter Temperatur

In 900 g voll entsalztem Wasser (VE-Wasser) werden 28,500 g NaCl, 0,220 g NaHCO₃, 4,066 g Na₂SO₄, 1,625 g CaCl₂ x 2 H₂O, 3,162 g MgCl₂ x 6 H₂O, 0,024 g SrCl₂ x 6 H₂O, 0, 721 g KCl gelöst und mit VE-Wasser auf 1 Liter aufgefüllt.

99,5 g dieser Lösung werden in einer 125 ml Weithalsflaschen aus NALGENE® FEP (Tetrafluorethylen-Hexafluorpropylen-Copolymer; Thermo Scientifc) vorgelegt und 0,5 g der zu prüfenden Dispersion hinzugegeben und durch Schütteln homogenisiert. Das Gemisch wird bei 60°C im Trockenschrank gelagert und das Auftreten eines Niederschlages visuell kontrolliert.

### Einsatzstoffe

### Silicium-Aluminium-Mischoxid

A: AEROSIL® MOX 170, Evonik Industries
   Das Pulver weist folgende Eigenschaften auf:
   99 Gew.-% Siliciumdioxid, 1 Gew.-% Aluminiumoxid. Die BET-Oberfläche beträgt 173 m²/g. (Al₂O₃/SiO₂)ₜₜₗ/ (Al₂O₃/SiO₂)_{Oberfläche} = 0,9.
B: Gemäß EP-A-995718, Beispiel 1. Das Pulver weist folgende Eigenschaften auf:
   99,7 Gew.-% SiO₂, 0,27 Gew.-% Al₂O₃-Anteil. Der Aluminiumoxidanteil ist homogen verteilt. Die BET-Oberfläche beträgt 55 m²/g.

AERODISP® W 7512 S, Evonik Industries, ist eine saure, niedrigviskose, wässrige Kieselsäure-Dispersion mit einem Feststoffgehalt von 12 %. Der zugrundeliegende Feststoff ist AEROSIL® 200, Evonik Industries, eine pyrogene Kieselsäure mit einer BET-Oberfläche von 200 m²/g.

AERODISP® W 7520 N, Evonik Industries, ist eine mit Natronlauge stabilisierte, niedrigviskose, wässrige Kieselsäure-Dispersion mit einem Feststoffgehalt von 20%. Der zugrundeliegende Feststoff ist AEROSIL® 200, Evonik Industries, eine pyrogene Kieselsäure mit einer BET-Oberfläche von 200 m²/g.

AERODISP® W 630, Evonik Industries, ist eine wässrige Aluminiumoxid-Dispersion mit einem pH-Wert von 3 - 5 und einem Feststoffgehalt von 30%. Der zugrundeliegende Feststoff ist

AEROXIDE® Alu C, Evonik Industries, eine pyrogenes Aluminiumoxid mit einer BET-Oberfläche von 100 m²/g.

LUDOX® SM 30, Grace, ist eine wässerige, mit NaOH stabilisierte, kolloidale Silicadispersion mit einer Partikelgröße von 8 nm und einem Gehalt an SiO₂ von 30 Gew.-%.

LUDOX® HS 40, Grace, ist eine wässerige, mit NaOH stabilisierte, kolloidale Silicadispersion mit einer Partikelgröße von 12 nm und einem Gehalt an SiO₂ von 40 Gew.-%.

LUDOX® CL, Grace, ist eine wässerige Dispersion von mit AI umhüllten, kolloidalem Silica mit 22 nm Partikelgröße. Der pH ist 3,5 - 4,5, der Feststoffgehalt 39 - 43 Gew.-%.

### Mittel zur Oberflächenmodifizierung

OM1: 2-[Methoxy(polyethyleneoxy)₆₋₉propyl] trimethoxysilan
OM2: Hydrolysat aus 3-Glycidyloxypropyl-Trimethoxysilan gemäß Beispiel 1, EP-A-832911 Wasser: es handelt es sich um vollentsalztes Wasser; Natronlauge: 25 Gew.-% NaOH; Salzsäure: 20 Gew.-% HCl

### Beispiel 1 (erfindungsgemäß): Silicium-Aluminium-Mischoxid A und OM1

### Herstellung einer 20 prozentigen Dispersion von Silicium-Aluminium-Mischoxid A

In einem 100 I Edelstahl-Ansatzbehälter werden 37 kg Wasser vorgelegt. Anschließend werden unter Scherbedingungen (Ystral Conti-TDS 3 (Statorschlitze: 4 mm Kranz und 1 mm Kranz, Rotor/Stator-Abstand ca. 1 mm) zunächst 10 kg AEROSIL® MOX 170 eingesaugt. Die restlichen 5 kg wurden schrittweise zu je ca. 1 kg eingesaugt. Nach Beendigung der Zugabe wurde bei 3000 U/min 30 min lang nachgeschert. Um noch restliche Anteile grober Partikel zu vermahlen wurde diese Vordispersion in zwei Durchgängen durch die Hochenergiemühle Sugino Ultimaizer HJP-25050 bei einem Druck von 2500 bar und Diamantdüsen von 0,25 mm Durchmesser geführt und dadurch intensiv weiter vermahlen.

Der mittlere, durch statische Lichtstreuung (LA- 950, Horiba Ltd., Japan) ermittelte Partikeldurchmesser d₅₀ ist 112 nm.

Zu 40 g dieser Dispersion werden langsam und Rühren 9,63 g OM1 zugegeben. Dabei tritt zuerst ein Viskositätsanstieg auf, der aber bei weiterer Zugabe wieder abnimmt. Nun wird mit Natronlauge unter Rühren ein pH von 11 eingestellt und die Mischung auf 90°C erwärmt. Nach 10 Minuten bei 90°C wird auf Raumtemperatur abkühlen lassen und mit Salzsäure ein pH von 9 eingestellt.

d₅₀ = 121 nm; Stabilität in Referenzlösung bei 60°C: 9 Monate.

### Beispiel 2 (erfindungsgemäß): Silicium-Aluminium-Mischoxid B und OM2

Entsprechend Beispiel 1 wird eine 20 prozentige Dispersion von Silicium-Aluminium-Mischoxid B hergestellt. Der mittlere, durch statische Lichtstreuung (LA- 950, Horiba Ltd., Japan) bestimmte Partikeldurchmesser d₅₀ ist 82 nm.

Zu 40 g dieser Dispersion werden langsam und Rühren 6,82 g DYNALYSAN® HYDROSIL 2926 gegeben. Die Mischung weist nun einen pH von 2,84 auf. Anschließend wird der pH-Wert mit Natronlauge auf pH 11 gestellt und 10 Min auf 90 °C erhitzt. Nach dem Abkühlen wird der pH-Wert mit Salzsäure auf 9 gestellt.

d₅₀ = 93 nm; Stabilität in Referenzlösung bei 60°C: 1 Monat

### Beispiel 3 (Vergleich)

Zu 67g AERODISP® W 7512 S werden langsam und unter Rühren 11,3 g OM1 gegeben. Es tritt zuerst ein Viskositätsanstieg auf der aber bei weiterer Zugabe wieder abnimmt. Nun wird mit Natronlauge unter Rühren ein pH von 11 eingestellt und die Mischung auf 90°C erwärmt. Nach 10 Minuten bei 90°C wird abgekühlt und mit Salzsäure ein pH von 9 eingestellt.

d₅₀ = 109 nm; Stabilität in Referenzlösung bei 60°C: 1 Tag

### Beispiel 4 (Vergleich)

Zu 40 g AERODISP® W 7520 N werden langsam und unter Rühren 11,3 g OM1 zugegeben. Nun wird mit Natronlauge unter Rühren ein pH von 11 eingestellt und die Mischung auf 90°C erwärmt. Nach 10 Minuten bei 90°C wird abgekühlt und mit Salzsäure ein pH von 9 eingestellt.

d₅₀ = 99 nm; Stabilität in Referenzlösung bei 60°C: 1 Tag

### Beispiel 5 (Vergleich)

Zu 100 g einer mit Wasser auf 10 Gew.-% verdünnten LUDOX® 30 SM Dispersion werden bei 80°C innerhalb von 3 Stunden 4,3 g OM1 unter Rühren hinzu getropft. Es wird bei 80°C 6 Stunden weiter gerührt.

### Stabilität in Referenzlösung bei 60°C: 1 Tag

### Beispiel 6 (Vergleich)

Zu 249 g LUDOX® HS 40 werden 30 g OM1 gegeben. Die Dispersion wird auf 80°C erhitzt und bei dieser Temperatur 16 Stunden gehalten.

### Stabilität in Referenzlösung bei 60°C: 1 Tag

### Beispiel 7 (Vergleich)

26,7g LUDOX® CL werden mit 13,3 g Wasser auf 20 % verdünnt. Zu diesem Sol werden langsam und unter Rühren 13,0 g OM1 zugegeben. Nun wird mit Natronlauge unter Rühren ein pH von 11 eingestellt und die Mischung auf 90°C erwärmt. Nach 10 Minuten bei 90°C wird abgekühlt und mit Salzsäure ein pH von 9 eingestellt.

### Stabilität in Referenzlösung bei 60°C: 2 Tage

### Beispiel 8 (Vergleich)

26,7g AERODISP® W 630 werden mit 13,3 g Wasser auf 20 % verdünnt. Zu dieser Dispersion werden langsam und unter Rühren 5,67 g OM1 zugegeben Nun wird mit Natronlauge unter Rühren ein pH von 11 eingestellt und die Mischung auf 90°C erwärmt. Nach 10 Minuten bei 90°C wird abgekühlt und mit Salzsäure ein pH von 9 eingestellt.

### Stabilität in Referenzlösung bei 60°C: 1 Tag

Die erfindungsgemäßen Dispersionen der Beispiele 1 und 2 weisen eine sehr gute Salzstabilität bei erhöhten Temperaturen auf. Diese Stabilität ist bei den Vergleichsbeispielen 3 - 8 nicht gegeben. In den Beispielen 3 und 4 werden pyrogene Kieselsäuren, in den Beispielen 5 und 6 kolloidale Silicasole, in Beispiel 7 ein mit AI umhülltes Silicasol und in Beispiel 8 ein pyrogenes Aluminiumoxid anstelle des Silicium-Aluminium-Mischoxidpulvers eingesetzt.

### Beispiel 9 (erfindungsgemäß): Redispergierbares Pulver

Eine entsprechend Beispiel 1 hergestellte Dispersion wird mit Hilfe eines Mini Spray Dryer B-290 der Firma BÜCHI Labortechnik GmbH unter Verwendung von Stickstoff als Heißgasmedium ein Pulver erzeugt das sich einfach wieder redispergieren läßt: Durch Einrühren mit Hilfe eines Magnetrührer erhält man ein d₅₀ von 155 nm, mit einem Dissolver nach 5 Minuten bei 2000 U/min ein d₅₀ von136 nm und mit einem ULTRA-TURRAX® T 25, IKA®-Werke GmbH & CO. KG, nach einer Minute bei 9000 U/min ein d₅₀ von 130 nm.

## Patentansprüche

1. Wässerige Dispersion enthaltend ein oberflächenmodifiziertes, pyrogenes hydrophiles Mischoxidpulver umfassend Silicium und Aluminium, **dadurch gekennzeichnet, dass**
a) die Oberfläche der Partikel Si- und Al-Atome und
b) die Oberflächenmodifizierung ein Si-Atom aufweist, welches über ein C-Atom an einen Kohlenwasserstoffrest gebunden ist und
c) der Kohlenstoffgehalt des oberflächenmodifizierten Mischoxidpulvers 3 - 25 Gew.-% beträgt und
d) das oberflächenmodifizierte Mischoxidpulver einen mittleren Partikeldurchmesser d₅₀ in der wässerigen Dispersion von 40 - 200 nm hat.

2. Wässerige Dispersion nach Anspruch 1, **dadurch gekennzeichnet, dass** bei der das Si-Atom, welches über ein C-Atom an einen Kohlenwasserstoffrest gebunden ist, -Si-O-Al-Bindungen bildet, wobei das Al-Atom Bestandteil der Partikeloberfläche ist.

3. Wässerige Dispersion nach den Ansprüchen 1 oder 2, **dadurch gekennzeichnet, dass** das das Gewichtsverhältnis Al₂O₃/SiO₂ im oberflächenmodifizierten Mischoxidpulver 0,1:99,9 - 5:95 ist.

4. Wässerige Dispersion nach den Ansprüchen 1 bis 3, **dadurch gekennzeichnet, dass** (Al₂O₃/SiO₂)_{Oberfläche} / (Al₂O₃/SiO₂)ₜₜₗ 0,1 - 10 ist.

5. Wässerige Dispersion nach den Ansprüchen 1 bis 4, **dadurch gekennzeichnet, dass** das oberflächenmodifizierte Mischoxidpulver überwiegend oder vollständig in Form von aggregierten Partikeln vorliegt.

6. Wässerige Dispersion nach den Ansprüchen 1 bis 5, **dadurch gekennzeichnet, dass** der Kohlenwasserstoffrest, der über ein C-Atom an ein Si-Atom gebunden ist, durch ein oder mehrere Heteroatome unterbrochen ist.

7. Wässerige Dispersion nach den Ansprüchen 1 bis 6, **dadurch gekennzeichnet, dass** die Oberflächenmodifizierung aus der Gruppe bestehend aus Si-(CH₂)ₙ-Yₘ-R ausgewählt ist, wobei Si das Si-Atom ist, welches über ein C-Atom an einen Kohlenwasserstoffrest gebunden ist, und
n = 1, 2, 3 und m= 0, 1;
für den Fall, dass m = 1 ist:
R = -H, -CH₃,-C₂H₅, -OH, -OCH₃, -OC₂H₅, -C(=O)OCH₃, -C(=O)OC₂H₅, -O-C(=O)CH₃,-O-C(=O)CH=CH₂, -O-C(=O)CH=CH(CH₃), -C(=O)CH₃, -C(=O)H, NH₂; 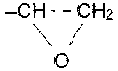 oder ein Gemisch der vorgenannten Reste R
und für den Fall, dass m=0 ist, R den vorgenannten Resten entspricht, jedoch ohne -H, -CH₃,-C₂H₅.
Y = -(OCR¹R²-CR³R⁴)ₒ-, o = 1 - 30, R¹, R²,R³, R⁴ = unabhängig voneinander H oder CH₃, besonders bevorzugt o = 5 - 15 und R¹, R²,R³, R⁴ = H;
-(OCR¹R²-CR³R⁴-CR⁵R⁶)ₚ- , p = 1 - 30, R¹, R²,R³, R⁴,R⁵, R⁶= unabhängig voneinander H oder CH3, -NHCH₂CH₂O-, -NH(CH₂)₂NH(CH₂)₂-, -NH(CH₂)₂NH(CH₂)₂- oder ein Gemisch der vorgenannten Reste Y.

8. Wässerige Dispersion nach den Ansprüchen 1 bis 7, **dadurch gekennzeichnet, dass** in der wässerigen Dispersion der Anteil an Wasser 50 - 90 Gew.-% und an oberflächenmodifiziertem Mischoxidpulver 10 - 50 Gew.-% beträgt.

9. Wässerige Dispersion nach den Ansprüchen 1 bis 8, **dadurch gekennzeichnet, dass** der pH-Wert der flüssigen Phase der wässerigen Dispersion 8 bis 12 ist.

## Claims

1. Aqueous dispersion comprising a surface-modified, fumed hydrophilic mixed oxide powder comprising silicon and aluminium, **characterized in that**
a) the surface of the particles has Si and Al atoms and
b) the surface modification has an Si atom bonded to a hydrocarbon radical via a C atom and
c) the carbon content of the surface-modified mixed oxide powder is 3 - 25% by weight and
d) the surface-modified mixed oxide powder has a median particle diameter d₅₀ of 40 - 200 nm in the aqueous dispersion.

2. Aqueous dispersion according to Claim 1, **characterized in that** in which the Si atom, which is bonded to a hydrocarbon radical via a C atom, forms -Si-O-Al bonds, the Al atom being a constituent of the particle surface.

3. Aqueous dispersion according to Claims 1 or 2, **characterized in that** the Al₂O₃/SiO₂ weight ratio in the surface-modified mixed oxide powder is 0.1:99.9 - 5:95.

4. Aqueous dispersion according to Claims 1 to 3, **characterized in that** (Al₂O_{3/}SiO₂)_{surface}/(Al₂O_{3/}SiO₂) ttl is 0.1 - 10.

5. Aqueous dispersion according to Claims 1 to 4, **characterized in that** the surface-modified mixed oxide powder is predominantly or completely in the form of aggregated particles.

6. Aqueous dispersion according to Claims 1 to 5, **characterized in that** the hydrocarbon radical bonded to an Si atom via a C atom is interrupted by one or more heteroatoms.

7. Aqueous dispersion according to Claims 1 to 6, **characterized in that** the surface modification is selected from the group consisting of Si-(CH₂)ₙ-Yₘ-R, wherein Si is the Si atom bonded to a hydrocarbon radical via a C atom, and
n = 1, 2, 3 and m = 0, 1;
in the case that m = 1:
R = -H, -CH₃, -C₂H₅, -OH, -OCH₃, -OC₂H₅, -C(=O)OCH₃, -C(=O)OC₂H₅, -O-C(=O)CH₃, -O-C(=O)CH=CH₂, -O-C (=O) CH=CH (CH₃) , -C(=O)CH₃, -C(=O)H, NH₂, 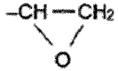 or a mixture of the aforementioned radicals R
and in the case that m = 0, R corresponds to the aforementioned radicals but not -H, -CH₃, -C₂H₅,
Y = - (OCR¹R²-CR³R⁴)ₒ-, o = 1 - 30, R¹, R², R³, R⁴ = each independently H or CH₃,
particularly preferably o = 5 - 15 and R¹, R², R³, R⁴ = H;
-(OCR¹R²-CR³R⁴-CR⁵R⁶)ₚ-, p = 1 - 30, R¹, R², R³, R⁴, R⁵, R⁶ = each independently H or CH₃, -NHCH₂CH₂O-,-NH(CH₂)₂NH(CH₂)₂-, -NH(CH₂)₂NH(CH₂)₂- or a mixture of the aforementioned radicals Y.

8. Aqueous dispersion according to Claims 1 to 7, **characterized in that** in the aqueous dispersion the proportion of water is 50 - 90% by weight and the proportion of surface-modified mixed oxide powder is 10 - 50% by weight.

9. Aqueous dispersion according to Claims 1 to 8, **characterized in that** the pH of the liquid phase of the aqueous dispersion is 8 to 12.

## Revendications

1. Dispersion aqueuse contenant une poudre d'oxyde mixte modifié en surface, hydrophile pyrogène, comprenant du silicium et de l'aluminium, **caractérisée en ce que**
a) la surface des particules présente des atomes de Si et d'Al et
b) la modification de surface présente un atome de Si, qui est lié à un radical hydrocarboné par l'intermédiaire d'un atome de C et
c) la teneur en carbone de la poudre d'oxyde mixte modifié en surface est de 3 à 25 % en poids et
d) la poudre d'oxyde mixte modifié en surface possède un diamètre moyen de particule d₅₀ dans la dispersion aqueuse de 40 à 200 nm.

2. Dispersion aqueuse selon la revendication 1, **caractérisée en ce que** dans celle-ci l'atome de Si, qui est lié à un radical hydrocarboné par l'intermédiaire d'un atome de C, forme des liaisons -Si-O-Al-, l'atome d'Al étant un élément de la surface de particule.

3. Dispersion aqueuse selon la revendication 1 ou 2, **caractérisée en ce que** le rapport pondéral Al₂O₃/SiO₂ dans la poudre d'oxyde mixte modifié en surface est de 0,1:99,9 à 5:95.

4. Dispersion aqueuse selon les revendications 1 à 3, **caractérisée en ce que** (Al₂O_{3/}SiO₂)_{surface}/(Al₂O_{3/}SiO₂) total est de 0,1 à 10.

5. Dispersion aqueuse selon les revendications 1 à 4, **caractérisée en ce que** la poudre d'oxyde mixte modifié en surface est présente majoritairement ou totalement sous forme de particules agrégées.

6. Dispersion aqueuse selon les revendications 1 à 5, **caractérisée en ce que** le radical hydrocarboné, qui est lié à un atome de Si par l'intermédiaire d'un atome de C, est interrompu par un ou plusieurs hétéroatomes.

7. Dispersion aqueuse selon les revendications 1 à 6 **caractérisée en ce que** la modification de surface est choisie dans le groupe constitué par Si(CH₂)ₙ-Yₘ-R, Si étant l'atome de Si, qui est lié à un radical hydrocarboné par l'intermédiaire d'un atome de C,
et
n = 1, 2, 3 et m = 0, 1 ;
dans le cas où m = 1 :
R = -H, -CH₃, -C₂H₅, -OH, -OCH₃, -OC₂H₅, -C(=O) OCH₃, -C(=O)OC₂H₅, -O-C(=O)CH₃, -O-C(=O)CH=CH₂, -O-C(=O)CH=CH(CH₃), -C(=O)CH₃, -C(=O)H, NH₂ ; 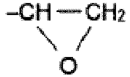 ou un mélange des radicaux R mentionnés précédemment
et dans le cas où m = 0, R correspond aux radicaux mentionnés précédemment, mais sans -H, -CH₃, -C₂H₅,
Y = - (OCR¹R²-CR³R⁴)ₒ-, o = 1 à 30, R¹, R², R³, R⁴ = indépendamment les uns des autres H ou CH₃, particulièrement préférablement o = 5 à 15 et R¹, R², R³, R⁴ = H ;
-(OCR¹R²-CR³R⁴-CR⁵R⁶)ₚ-, p = 1 à 30, R¹, R², R³, R⁴, R⁵, R⁶ = indépendamment les uns des autres H, CH₃, -NHCH₂CH₂O-, -NH(CH₂)₂NH(CH₂)₂-, -NH(CH₂)₂NH(CH₂)₂- ou un mélange des radicaux Y mentionnés précédemment.

8. Dispersion aqueuse selon les revendications 1 à 7, **caractérisée en ce que** dans la dispersion aqueuse la proportion d'eau est de 50 à 90 % en poids et la proportion de poudre d'oxyde mixte modifié en surface est de 10 à 50 % en poids.

9. Dispersion aqueuse selon les revendication 1 à 8, **caractérisée en ce que** la valeur de pH de la phase liquide de la dispersion aqueuse est de 8 à 12.
